# EUROPEAN PATENT SPECIFICATION

(11) **EP 0 991 761 B1**
(45) Date of publication and mention of the grant of the patent: **16.01.2008**
(21) Application number: 97936881.8
(22) Date of filing: 21.08.1997
(51) Int. Cl.: C12N 15/31, C12N 1/20, A61K 39/02, A61K 39/095, C07K 14/22

(54) **NOVEL MUTANTS OF GRAM NEGATIVE MUCOSAL BACTERIA AND APPLICATION THEREOF IN VACCINES**
NEUE MUTANTEN DER GRAM-NEGATIVEN MUKOSALEN BAKTERIEN UND IHRE VERWENDUNG ALS IMPFSTOFFE
NOUVEAUX MUTANTS DE BACTERIES DES MUQUEUSES GRAM NEGATIVES ET LEUR APPLICATION DANS DES VACCINS

(43) Date of publication of application: 12.04.2000
(73) Proprietor: DE STAAT DER NEDERLANDEN VERTEGENWOORDIGD DOOR DE MINISTER VAN WELZIJN, VOLKSGEZONDHEID EN CULTUUR, NL-2280 HK Rijswijk (NL)
(72) Inventor: VAN DER LEY, Peter, André, NL-3572 DJ Utrecht (NL); STEEGHS, Liana, Juliana, Josephine, Margret, NL-3511 NP Utrecht (NL)
(74) Representative: van Westenbrugge, Andries
(86) International application number: PCT/NL1997/000474
(87) International publication number: WO 1999/010497

(56) References cited:
- EP-A- 0 624 376
- WO-A-97/19688
- WO-A-97/25061
- STEEGHS ET AL: "ISOLATION AND CHARACTERIZATION OF THE NEISSERIA MENINGITIDIS LPXD-FABZ-LPXA GENE CLUSTER INVOLVED IN LIPID A BIOSYNTHESIS" GENE, vol. 190, no. 2, 6 May 1997, pages 263-270, XP002064116 cited in the application
- ODEGAARD ET AL: "SHORTENED HYDROXYACYL CHAINS ON LIPID A OF ESCHERICHIA COLI CELLS EXPRESSING A FOREIGN UDP-N-ACETYLGLUCOSAMINE O-ACYLTRANSFERASE" THE JOURNAL OF BIOLOGICAL CHEMISTRY, vol. 272, no. 32, 8 August 1997, pages 19688-19696, XP002064117
- RAETZ ET AL: "BIOCHEMISTRY OF ENDOTOXINS" ANNUAL REVIEWS OF BIOCHEMISTRY, vol. 59, 1990, pages 129-170, XP002064118 cited in the application

## Description

### Summary of the invention

We found that contrary to previous findings with *E. coli* it is possible to inactivate the early stage of lipid A synthesis of mucosal gram negative bacteria without compromising cell viability. In particular the *lpxA* gene in *Neisseria meningitidis* was mutated without compromising cell viability. The resulting *lpxA* knockout mutants were found to be completely LPS-deficient. The major outer membrane proteins (OMPs) were detected in normal amounts. Also, An outer membrane could be discerned in electron micrographs of ultrathin sections. To our knowledge, this was the first instance of a viable Gram-negative bacterial mutant completely lacking in LPS.

The finding provides important implications for our understanding of structure and biogenesis of the outer membrane. On a practical level, the availability of LPS-deficient mutants of pathogenic mucosal bacteria such as *N. meningitidis* opens up new avenues to vaccine development. It enables easy isolation of endotoxin-free purified proteins, outer membranes or even whole-cell preparations for use in immunisation.

### Background information

Lipopolysaccharide (LPS) constitutes the outer monolayer of the outer membrane of Gram-negative bacteria. As such it forms an important component of the outer membrane and has been considered relevant for vaccine purposes (Verheul et al, 1993). The membrane-anchoring lipid A part is responsible for the well-known endotoxin activity of the molecule (Zähringer et al., 1994).

Such endotoxin activity is undesirable in vaccines. Currently some preparations to be used in vaccines are subjected to rigorous, time consuming and costly purification procedures in order to remove this endotoxin activity prior to their being suitable for use as a vaccine. This allows higher closes due to reduced toxicity. However, drastic purification methods can easily lead to denaturation of protein antigens which need to retain their native conformation in order to induce an appropriate immune response. To date Group A and C polysaccharide vaccines are available which have been rendered substantially free of lipo-polysaccharide by means of purification. To date however no whole cell vaccines substantially free of LPS nor OMP vaccines substantially free of LPS have been produced. The following references provide details of processes used to date in order to avoid LPS in pharmaceutical products Akers (1985), Gabler (1987) and the European Pharmacopoeia, 2nd edition "test for non-pyrogenicity". Specifically WHO Tech. Rep. Ser 594:50 1976 deals with the requirements for a meningococcal polysaccharide vaccine.

Mutants with defects in LPS biosynthesis have been described for many bacterial species however none of these have been considered as candidates for a vaccine free of the endotoxic LipidA. All viable mutants retain a minimal lipid A - KDO structure which is the first part to be synthesised (Raetz, 1990) in LPS synthesis. Thus they would not be suitable to overcome the above-mentioned problem facing vaccine producers. Above all, only conditionally lethal mutations have been reported for genes involved in early steps of lipid A biosynthesis in *Escherichia coli* (Raetz 1990). These mutants have a mutation in genes involved in early steps of lipid A biosynthesis. This finding suggested that this part of the LPS molecule is in fact essential for bacterial growth. As such this finding would be considered dissuasive by persons skilled in the art of producing vaccines of mutating genes associated herewith as the resulting mutant would not grow.
Inhibitors of lipid A biosynthesis have also been found to lead to rapid loss of cell viability in *E. coli* and several other bacteria (Onishi et al, 1996) thus supporting the above-mentioned hypothesis concerning the essential nature of lipidA biosynthesis.

In addition models for biogenesis of OMPs have been proposed in which their correct folding and targeting is dependent on LPS (Sen and Nikaido, 1991; Reid et al., 1990; Laird et al., 1994; de Cock and Tommassen, 1996).

WO 97/25061 discloses mutants of gram-negative bacteria having a form of LPS deficient in levels of myristic acid moiety, in which the *lpxF* gene is inhibited.

WO 97/19688 describes mutants of gram-negative bacteria producing less toxic LPS as a result of a mutation in the *htrB* gene.

We previously cloned the *lpxA* gene from *Neisseria meningitidis* which encodes the enzyme UDP-GlcNAc acyltransferase required for the first step in lipid A biosynthesis (Steeghs et al. 1997). While attempting to alter the fatty acyl specificity of this enzyme by constructing an *E*. *coli-N. meningitidis* hybrid *lpxA* gene, we made the unexpected discovery forming the basis of the subject invention.

### Detailed description of the invention.

The isolation of the *N. meningitidis lpxA* gene involved in lipid A biosynthesis has recently been reported (Steeghs et al., 1997). The deduced amino acid sequence of the LpxA protein showed homology to the *E.coli* acyltransferase responsible for adding the *0*-linked 3-OH myristoyl chain to UDP-N-acetylglucosamine, which is the first committed step in the lipid A biosynthetic pathway (Anderson and Raetz, 1987 ; Coleman and Raetz, 1988). Based on this homology and a comparison of the *E.coli* and *N.meningttidts* lipid A structures it is expected that the meningococcal *lpxA* gene encodes an acyltransferase with 3-0H lauroyl specificity (Kulshin et al., 1992). The basis of the different fatty acid specificity might conceivably be located in the characteristic hexapeptide repeat motif of these acyltransferases which has been determined to play a crucial role in the folding of the *E.coli* protein (Vuorio et al., 1994; Raetz and Roderick, 1995). In an attempt to verify this hypothesis we constructed a hybrid *lpxA* gene in which the meningococcal N-terminal β-helix domain containing the hexapeptide repeat motif was replaced by the corresponding part of *E.coli lpxA.* followed by transformation and allelic replacement of this construct to *N. meningiti*dis H44/76. The experimental data for this are provided in the examples (in particular example 1).

The results demonstrated that strain H44/76[pHBK30] is a viable LPS-deficient mutant. The most likely explanation for this surprising discovery seemed to be that the hybrid *lpxA* gene had become inactive, either because of disrupted transcription/translation in our construct, or else because the hybrid protein as expressed had lost its enzymatic activity. To discern this, we constructed an *lpxA* knockout mutant. The results demonstrated once more that blocking of the lipid A biosynthesis pathway in *N. meningitidis* strain H44/76 leads to viable LPS-deficient mutants.

This is the first report of a viable Gram-negative bacterial mutant completely deficient in LPS. It has the following implications:
(1) Surprisingly (in view of the above mentioned view of the essential nature of lipidA biosynthesis for cell viability), it is possible for some gram negative bacteria to make an outer membrane without any LPS yet remain viable. Although our results do not exclude an involvement of LPS in the OMP forming process, they do demonstrate that it obviously cannot be essential. It should be very interesting to study the structure of the outer membrane in the *lpxA* mutant in more detail,
(2) In *E.coli,* all mutations affecting the early steps of lipid A biosynthesis that have been described are lethal when expressed. The fact that this is not the case in Meningococci opened up the question which organism is typical in this respect, and what causes this difference. Conceivably, it is related to a different LPS-OMP interaction, which is also suggested by the observation that whereas deep-rough LPS mutants of *E. coli* and *Salmonella typhimurium* show a reduced expression of the major OMPs (Koplow and Goldfine. 1974; Ames et al., 1974), a comparable heptose-deficient *rfaC* mutant of *N.meningitidis* was found to have normal expression of the class 1 and 3 porins (Hamstra and van der Ley, unpublished).
   We postulate that mucosal gram negative bacteria can in an analogous manner be mutated thereby becoming free of endotoxic LPS. Subsequently enabling development of LPS free whole cell or acellular vacines such as OMP vaccines. The basis for this postulation is found in the knowledge available to the skilled person concerning the Lipid a biosynthesis in mucosal gram negative bacteria. Figure 6 e.g. as derived from Raetz 1990 provides a diagram of the early steps in lipid A biosynthesis. It reveals the requirement of *lpxA* and *lpxB* as enzymes required in the early biosynthesis. The enzyme *lpxD* is also known to be involved (Steeghs et al 1997). Knowledge of the nucleic acid sequences encoding these genes is available to the skilled person (Steeghs et al 1997). Subsequently mutating one or more of the genes encoding the enzymes involved in the early stages of LipidA biosynthesis is possible. Figure 6 shows the early stages; preferably the mutation will arise such that no stage leading past the *lpxB* stage is reached as these products already closely resemble Lipid A structure. Preferably the mutation will arise as early as possible in the biosynthesis pathway. In most cases the genes encoding *lpxA, lpxB* and *lpxD* are clustered. Steeghs et al provides references disclosing such details for *Escherichia coli, Salmonella typhimurium, Yersinia entereocolitica, Haemophilus influenzae* and *Rickettsia rickettsii.* Knowledge of the sequences of these microorganisms is thus available to the person skilled in the art and homologous sequences in other organisms can be found. Both *lpxA* and *lpxD* contain a characteristic hexapeptide repeat structure [(I.V.L)GXXXX]ₙ. The *lpxB* gene is generally cotranscribed with *lpxA* and as such can also be readily found. The cluster also comprises the *fabZ* gene which can also be used to ascertain the location of the gene cluster involved in Lipid A biosynthesis (Steeghs et al 1997). Steeghs et al provide the genbank number under which the *N.meningitidis* sequence data concerning the Lipid A biosynthesis gene cluster is available i.e. U79481.
   There are two possibilities to mutate one or more genes associated with LipidA biosynthesis. Such mutation can be either such that enzyme is produced in an inactive form or such that the gene is mutated such that it is not expressed i.e. thereby forming a socalled knockout mutant. The manners in which this objective can be achieved are numerous and will be readily available to a person skilled in the art of genetic engineering. Clearly by way of example of such a manner the analogous procedure to that employed in the examples can be used for different microorganisms and/or different enzymes known to be involved in the early stages of Lipid A biosynthesis. The principal of preparing knockout mutants in general is well known and can be applied to any of the genes encoding enzymes active in the early stages of Lipid A biosynthesis.
   The subject invention comprises the mutants described above. In addition it comprises new vaccines made from such organisms or component parts thereof. Such new vaccines are free of Lipid A. Such vaccines can in fact be completely free of either active or inactive Lipid A. As a consequence the vaccines are free of LPS. The Limulus test can be applied as described herein to ascertain that a preparation is in fact free of LPS.
   Thus a vaccine against a gram negative mucosal bacterium said vaccine being substantially free of LPS, wherein substantially free can be ascertained by the Limulus test, said vaccine comprising a microorganism according to the invention as disclosed above as active component falls within the scope of the invention. This is a so called whole cell vaccine. In addition a vaccine against a gram negative mucosal bacterium comprising one or more components of the aforementioned microorganism which is also substantially free of LPS as defined above is covered by the invention. In particular an OMP comprising vaccine substantially free of LPS as defined above is covered by the invention.
   A method of producing a vaccine against gram negative mucosal bacteria employing a microorganism according to the invention and/or parts derived therefrom as active component in a manner known per se for producing whole cell or acellular vaccines is covered by the invention as are the products of said method. The vaccines according to the invention will preferably be further characterised by the presence of an adjuvant to enhance the immunogenic activity thereof. A number of adjuvants commonly used in vaccines are known. Any of these can be suitably applied. Any dosage form and additional components commonly used for vaccines, in particular meningococcal vaccines is suitable for the subject invention.
   Particularly suited target microorganisms are diplococci and *Bordetella pertussis.* The diplococci comprise meningococci and gonococci. Examples of each category are *N.meningitidis and N. gonorrhoeae.* Numerous other organisms falling within this category are known from Bergeys Handbook of Determinative Bacteriology. These diplococci are structurally closely related and show the same gene structure. Both are interesting microorganisms from a vaccination view point as are a number of other microorganisms such as *Haemophilus influenzae* and *Moraxella catarrhalis.*
   Clearly, the construction of *lpxA* knockout mutants can be attempted in other bacterial species known to have LipidA in their lipopolysaccharide.
(3) The availability of LPS-deficient mutants will allow new approaches to vaccine development against *N.meningitidis* and the closely related pathogen *N.gonorrhoeae,* as well as any other bacteria as mentioned above for which such mutants can be made and isolated. First, it will become much easier to purify OMPs or other cell surface components without contaminating endotoxin. Secondly the role of LPS in meningococcal outer membrane vesicle vaccines, e.g. as adjuvant or in stabilising OMP conformation (Verheul et al., 1993; Nakano and Matsuura, 1995; Poolman, 1995), can now be unequivocally determined and possibly taken over by a less toxic compound. Thirdly the use of inactivated whole cell vaccines can be investigated using endotoxin-free mutants according to the invention such as the *lpxA* mutants. Finally, the possibility to use LPS-deficient strains as live attenuated vaccines now arises.

The exact nature of the invention will be further elucidated with the following examples.

### Example 1: Construction of an inactive lpxA gene in N.meningitidis

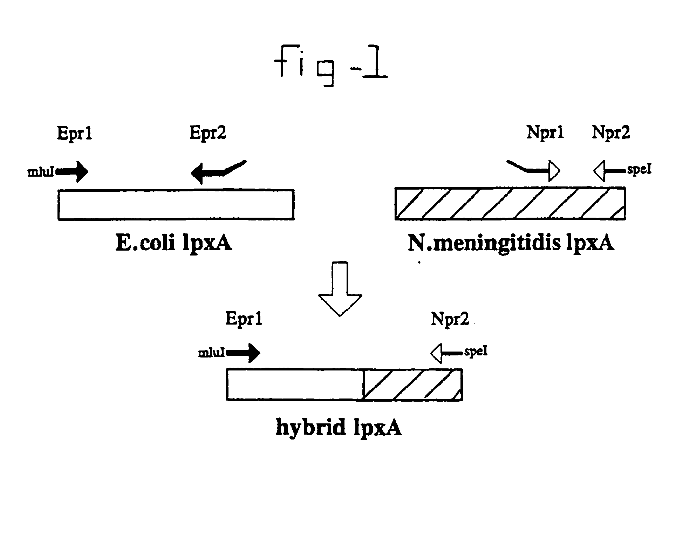

In two separate PCR reactions the *E.coli* and *N.meningitidis* part of the hybrid gene were amplified with the Epr1/Epr2 and Npr1/Npr2 primer, respectively (fig.1). The inside primers Epr2 and Npr1 were designed so that the ends of the products contain complementary sequences. These products were mixed, denatured and reannaeled in a second PCR in which the fused construct was amplified by the outside primers Epr1 and Npr2, having an *MluI* and *SpeI* site respectively (fig.1). The resulting PCR product was cloned and its sequence verified.

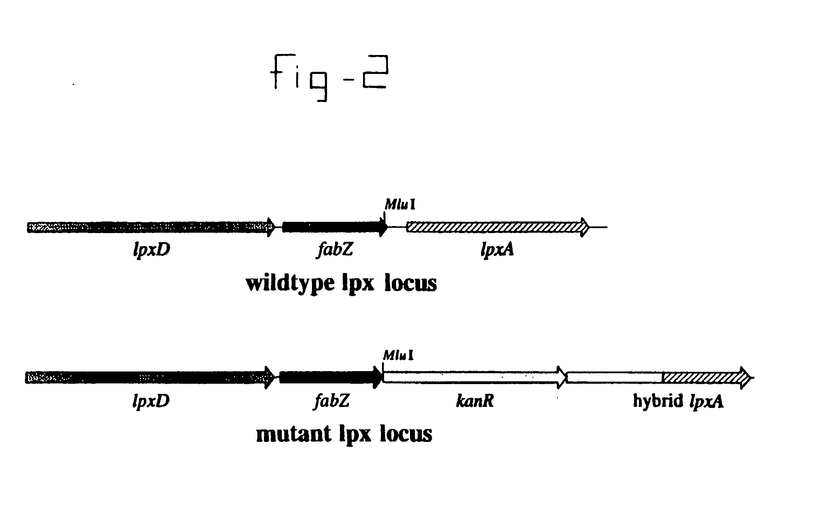

To test the activity of the hybrid *lpxA,* this gene was used to replace the original *lpxA* in the meningococcal chromosome (fig.2). For this purpose the 1.0 kb *MluI*/*SpeI* fragment carrying the wildtype *lpxA* gene in plasmid pLA19 (a pUC18 derivative with a 1.9 kb *lpxD-fabZ-lpxA* insert) was replaced by the similarly digested hybrid *lpxA* gene. Subsequently, a kanamycin-resistance cassette was ligated into the *MluI* site located directly upstream of *lpxA*, resulting in the plasmid pHBK30.

Transformation of *N.meningitidis* H44/76 with linearized pHBK30 yielded kanamycin-resistant colonies after 24 hours of incubation. These mutants died when transferred to fresh CC plates with kanamycin (100 µg/ml).

By reducing the kanamycin concentration and screening of the resulting colonies by PCR amplification of *lpxA* hybrid-specific fragments we finally succeeded in the isolation of viable *kanRº* H44/76[pHBK30] transformants in which the chromosomal *lpxA* gene had been replaced by the hybrid construct as shown in fig.2.

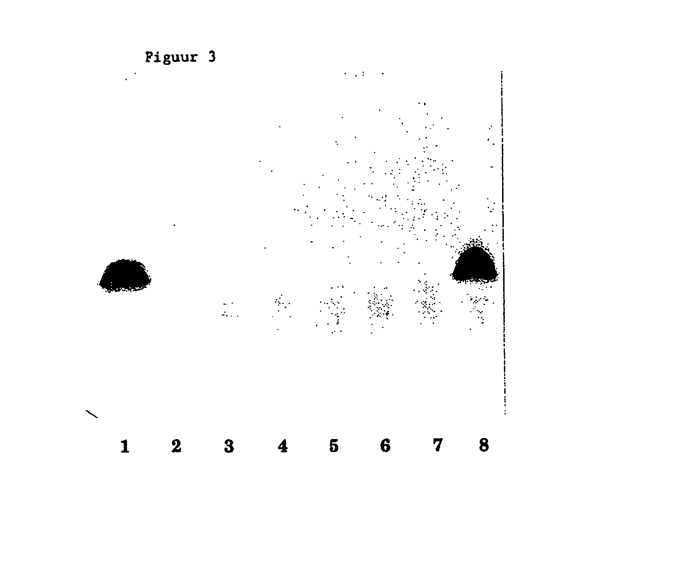

LPS of the H44/76[pHBK30] mutant and the wildtype strain was compared by Tricine-SDS-PAGE followed by a silver stain for carbohydrates (fig.3). Surprisingly, no LPS could be detected in the hybrid derivative by this method, even when higher amounts of cell lysates were loaded on the gel.

To get more insight into the structure of the outer membrane of H44/76[pHBK30] a panel of LPS and OMP specific mAbs was tested in a whole cell ELISA (Table 1). The mutant strain did not bind any of the LPS-specific mAbs, whereas the OMP-specific mAbs showed similar binding patterns for mutant and wildtype. This apparent OMP similarity was confirmed when OMCs of H44/76[pHBK30] and H44/76 were isolated and analysed by SDS-PAGE (fig.3). Both strains show equal amounts of the class 1. 3 and 4 OMP; in contrast to the wildtype, the mutant apparently also expresses a class 5 OMP.

Since LPS of H44/76[pHBK30] could not be detected with any of the methods described above, it became questionable whether it was present at all. Therefore, the mutant and wildtype strain were tested in a chromogenic Limulus (LAL) assay, with meningococcal medium as a negative control. This assay depends on activation of the clotting enzyme cascade in amoebocyte lysate prepared from the horseshoe crab and is capable of detecting picogram quantities of endotoxin. The results of the LAL assay on cell suspensions showed no significant endotoxin activity for H44/76[pHBK30] over meningococcal medium (0.3 and 1.7 EU/ml, respectively), in contrast to 21.7×10⁴ EU/ml for the wildtype.

Taken together, these results demonstrate that the initial attempt to replace the wildtype *lpxA* gene with the hybrid construct resulted in the isolation of what was apparently an LPS-deficient mutant. This was further confirmed by gas-chromatography/mass-spectrometry (GC-MS) analysis of fatty acids present in OMC preparations, which showed that the lipid A-specific 3-OH C12 was present only in trace amounts in the mutant. As this fatty acid is added in the first step of lipid A biosynthesis, its absence demonstrates that the mutant is truly LPS-deficient and not just making some incomplete precursor molecule with no antibody binding or LAL assay activity.

Although H44/76[pHBK30] is fully viable, a reduced growth rate compared to the wildtype strain was apparent. When grown overnight on GC agar plates, the mutant strain produced much smaller colonies; in liquid medium the doubling time during exponential growth was approximately 50% higher than in wildtype strain H44/76.

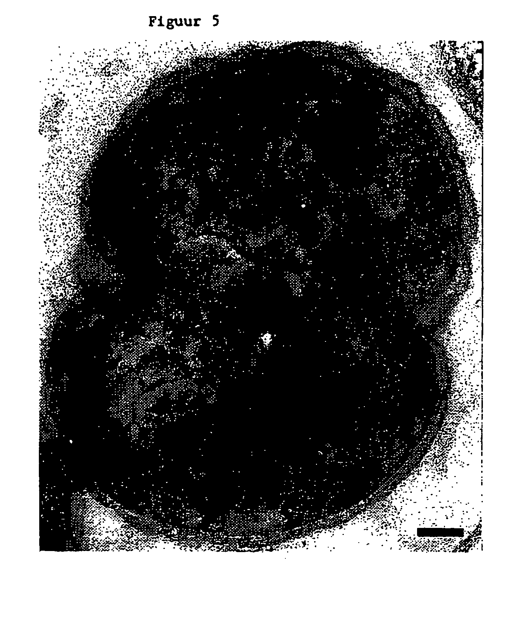

The morphology of H44/76[pHBK30] and its parent strain was examined by electron microscopy of ultrathin sections. In contrast to the wild type, cells of H44/76[pHBK30] were more heterogeneous in size and a significant fraction showed signs of lysis. However, the outer membrane could be clearly discerned in the LPS-deficient mutant (fig.5). In contrast to the somewhat "baggy" appearance in the wildtype, the outer membrane of the mutant showed a "tighter fit", possibly indicating a lowered rate of synthesis.

### Example 2: Construction of an lpxA knockout mutant

An *lpxA* knockout mutant of *N-meningitidis* was constructed by inserting a kanamycin-resistance cassette into the BstEII site located at position 293 within the *lpxA* gene of plasmid pLA21 (a pUC18 derivative with a 2.1 kb *lpxD-fabZ-lpxA* insert). The resulting plasmid pLAK33 was digested with *XbaI*/*SacI* and transformed to strain H44/76 with selection for kanamycin-resistance. As expected, the resulting colonies showed the same growth properties as the H44/76[pHBK30] mutant, indicating the lack of LPS. This was confirmed by a whole cell ELISA in which the *lpxA* knockout mutant did not bind any of the LPS-specific mAbs. These results demonstrated once more that blocking of the lipid A biosynthesis pathway in *N. meningitidis* strain H44/76 leads to viable LPS-deficient mutants.

### Detailed description of the methods and strains used in the examples.

Where no specific details are provided standard technology has been applied.

### Bacterial strains and plasmids

The *E.coli* strains NM522 and INVαF' were grown on LB medium at 37°C. The *N.meningitidis* strain H44/76 and its derivatives were grown at 37°C on GC medium base (Difco) supplemented with IsoVitaleX (Becton Dickinson) in a humid atmosphere containing 5% CO₂, or in liquid medium as described (van der Ley et al., 1993). For selection of meningococcal transformants (van der Ley et al., 1996) kanamycin was used in a concentration of 75-100 µg/ml. With *E.coli,* antibiotics were used in the following concentrations: ampicillin, 100 µg/ml; kanamycin. 100 µg/ml. For cloning of PCR fragments, the TA cloning kit with the vector pCRII (Invitrogen) was used. Another vector used was pUC18.

### Recombinant DNA techniques

Most recombinant DNA techniques were as described in Sambrook et al. (1989). Plasmid DNA was isolated using the pLASmix kit (Talent). The polymerase chain reaction (PCR) was performed on a Perkin Elmer GeneAmp PCR system 9600 with Taq polymerase. Sequence analysis was performed with an Applied Biosystems automatic sequencer on doublestranded plasmid DNA templates (isolated with Qiagen columns) and with a cycle sequencing protocol.

### LPS analysis

Tricine-sodium dodecyl sulphate polyacrylamide gel electrophoresis was performed in 4% stacking and 16% separating gels as described by Lesse et al. (1990). Proteinase K-treated, boiled bacterial cells were used as samples. The gels were run for 17 h at a constant current of 20 mA, and silver stained by the method of Tsai and Frasch (1982). The chromogenic LAL assay for endotoxin activity was performed using the QCL-1000 kit from BioWhittaker Inc. (Walkersville, MD, USA) according to the instructions of the manufacturer. Overnight cultures were diluted in meningococcal medium to an OD at 620 nm of 0.1, and serial dilutions of these stocks were used as samples in the LAL assay. For fatty acid analysis by GC-MS, OMC samples were acetylated for 3 h at 90°C in pyridine and acetic acid anhydride in order to completely dissolve the LPS. The samples were subsequently heated for 3 h at 65°C in tetrahydrofuran in the presence of LiAlH₄ to reduce the O-linked fatty acids to the free alcohols. These were derivatized to TMS-ethers for 1 h at 60°C with BSTFA + 1% TMCS in pyridine, and analyzed by GC-MS on an Autospec (Micromass, Manchester, UK) in the electron impact mode. The amount of 3-OH C12 in the samples was quantified using 2-OH C12 as internal standard.

### Characterization of OMP composition

Binding of mAbs specific for class 1. 3 and 4 OMPs and for the oligosaccharide part of immunotype L3 LPS was tested in a whole-cell ELISA (van der Ley et al., 1995, 1996). Isolation of OMCs by sarkosyl extraction and their analysis by SDS-PAGE were done as described previously (van der Ley et al., 1993).

### LEGENDS TO THE FIGURES

**Figure 1.** Construction of H44/76[pHBK30]. Two-step PCR mutagenesis leading to the hybrid *lpxA* gene, with *E.coli*-specific primers Epr1 (ACT-GACGCGTGTGATTGATAAATCCGC) seq. id. nr. 1 and Epr2 (GTACGGCGGCACCTCCTGCGCCACACCGGA) seq. id. nr. 2 and *N.meningitidis-*specific primers Npr1 (TCCGGTGTGGCGCAGGACGTGCCGCCCTAC) seq. id. nr. 3 and Npr2 (CGGCCGCTCTAGAACTAGTGGATCA) seq. id. nr. 4.
**Figure 2**. Construction of H44/76[pHBK30]. Replacement of the chromosomal *lpxD-fabZ-lpxA* locus with the pHBK30 insert, carrying in addition to the *E.coli-N.meningitidis* hybrid *lpxA* gene a *kanR* selection marker instead of the 99 bp region between the MluI site in *fabZ* and the start codon of *lpxA.*
**Figure 3**. SDS-PAGE analysis of H44/76[pHBK30]. Silver-stained Tricine-SDS-PAGE LPS gel of proteinase K-treated whole-cell lysates of H44/76 (lanes 1 and 8) and six independent kanamycin-resistant transformants with pHBK30 (lanes 2-7).

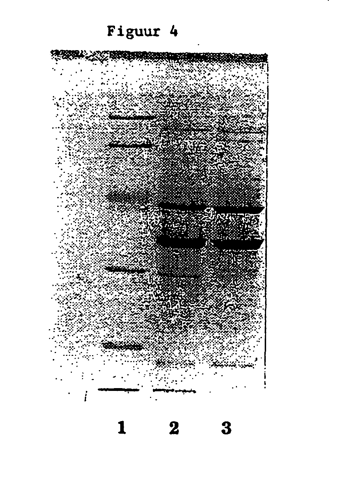

**Figure 4**. SDS-PAGE of OMC proteins from H44/76[pHBK30] (lane 2) and H44/76 wildtype (lane 3): lane 1 contains a molecular weight marker of 94. 67. 43. 30. 20.1 and 14.4 kDa.
**Figure 5**. Electron micrograph of an H44/76[pHBK30] thin section, showing the presence of the outer membrane in the absence of LPS.

### REFERENCES

Akers, M.J. Parenteral Quality Control. Chapter 2: Pyrogen Testing. Marcel Dekker, Inc. New York and Basel, 1985, pp. 79-142.
Ames. G. F.-L., Spudich, E.N. and Nikaido, H.: Protein composition of the outer membrane of Salmonella typhimurium: effect of lipopolysacharide mutations. J. Bacteriol. 117 (1974) 406-416.
Anderson, M.S and Raetz, C.R.H: Biosynthesis of lipid A precursors in Escherichia coli. A cytoplasmic acyltransferase that converts UDP-N-acetylglucosamine to UDP-3-0-(R-3-hydroxymyristoyl)-N-acetylglucosamine. J. Biol. Chem. 262 (1987) 5159-5169.
de Cock. H. and Tommassen, J.: Lipopolysaccharides and divalent cations are involved in the formation of an assembly-competent intermediate of outer-membrane protein PhoE of E.coli. EMBO J. 15 (1996) 5567-5573.
Coleman, J. and Raetz, C.R.H.: First committed step of lipid A biosynthesis in Escherichia coli: Sequence of the lpxA gen e. J. Bacteriol. 170 (1988) 1268-1274.
Gabler, F.R. Pyrogens, and the depyrogenation of solutions with ultrafiltration membranes. In: Meltzer. T.H. Filtration in the pharmaceutical industry. Marcel Dekker, Inc. New York and Basel, 1987. pp. 919-939.
Karnovsky. M.J.: Use of ferrocyanide-reduced osmium tetraoxide in electron microscopy. Proc. 11th Ann. Meeting Soc. Cell Biol. 51 (1971) 146.
Koplow. J. and Goldfine. H.: Alterations in the outer membrane of the cell envelope of heptose-deficient mutants of Escherichia coli. J. Bacteriol. 117 (1974) 527-543.
Kulshin, V.A.. Zähringer, U., Lindner, B., Frasch C.E., Tsai. C., Dimitriev, A. and Rietschel, E.T.: Structural characterization of the lipid A component of pathogenic Neisseria meningitidis. J. Bacteriol. 174 (1992) 1793-1800.
Laird, M.W., Kloser, A.W. and Misra, R.: Assembly of LamB and OmpF in deep rough lipopolysaccharide mutants of Escherichia coli K-12. J. Bacteriol. 176 (1994) 2259-2264. Lesse. A.J.. Campagnari. A.A., Bittner. W.E. and Apicella. M.A.: Increased resolution of lipopolysaccharides and lipooligosaccharides utilising tricine-sodium dodecyl sulfate-polyacrylamide gel electrophoresis. J. Immunol. Meth. 126 (1990) 109-117.
van der Ley, P., van der Biezen. J., Hohenstein, P., Peeters, C. and Poolman, J.T.: Use of transformation to construct antigenic hybrids of the class 1 outer membrane protein in Neisseria meningitidis. Infect. Immun. 61 (1993) 4217-4224.
van der Ley, P.. van der Biezen, J. and Poolman, J.T.: Construction of Neisseria meningitidis strains carrying multiple chromosomal copies of the porA gene for use in the production of a multivalent outer membrane vesicle vaccine. Vaccine 13 (1995) 401-407.
van der Ley. P., Kramer, M., Steeghs, L., Kuipers. B., Andersen, S.R., Jennings, M.P., Moxon, E.R. and Poolman, J.T.: Identification of a locus involved in meningococcal lipopolysaccharide biosynthesis by deletion mutagenesis. Mol. Microbiol. 19 (1996) 1117-1125.
Nakano. M. and Matsuura. M.: Lipid A. In: Stewart-Tull. D.E.S. (Ed.). The Theory and Practical Application of Adjuvants. John Wiley & Sons. 1995, pp. 315-335.
Onishi, H.R., Pelak. B.A., Gerckens, L.S. et al.: Antibacterial agents that inhibit lipid A biosynthesis. Science 274 (1996) 980-982.
Poolman. J.T.: Development of a meningococcal vaccine. Infect. Agents Dis. 4 (1995) 13-28.
Raetz. C.R.H.: Biochemistry of endotoxins. Annu. Rev. Biochem. 59 (1990) 129-170.
Raetz. C.R.H. and Roderick. S.L.: A left-handed parallel β helix in the structure of UDP-N-Acetylglucosamine acyltransferase. Science 270 (1995) 997-1000.
Reid. G., Hindennach. I. and Henning. U.: Role of lipopolysaccharide in assembly of Escherichia coli outer membrane proteins OmpA, OmpC and OmpF. J. Bacteriol. 172 (1990) 6048-6053.
Sambrook, J., Fritsch. E.F. and Maniatis, T.: Molecular Cloning: A Laboratory Manual. Cold Spring Harbor Laboratory Press, Cold Spring Harbor, New York. 1989.
Sen. K. and Nikaido. H.: Lipopolysaccharide structure required for in vitro trimerization of Escherichia coli OmpF porin. J. Bacteriol. 173 (1991) 926-928).
Steeghs. L., Jennings. M.P., Poolman. J.T. and van der Ley, P.: Isolation and characterization of the Neisseria meningitidis lpxD-fabZ-lpxA gene cluster involved in lipid A biosynthesis. Gene 190 (1997) 263-270.Tsai. C.M. and Frasch. C.E.: A sensitive silver stain for detecting lipopo-lysaccharides in polyacrylamide gels. Anal. Biochem. 119 (1982) 115-119.
Verheul. A.F.M., Snippe, H. and Poolman, J.T.: Meningococal lipopolysaccharides: Virulence factor and potential vaccine component. Microbiol. Rev. 57 (1993) 34-49.
Vuorio, R., Härkönen, T., Tolvanen, M. and Vaara, M.: The novel hexapeptide motif found in the acyltransferases LpxA and LpxD of lipid A biosynthesis is conserved in various bacteria. FEBS Lett. 337 (1994) 289-292.
Zähringer. U., Lindner, B. and Rietschel. E.Th.: Molecular structure of lipid A, the endotoxic center of bacterial lipopolysaccharides. In: Horton, D. (Ed.), Advances in Carbohydrate Chemistry and Biochemistry, vol.50. Academic Press Inc, San Diego, 1994, pp. 211-276.

## Claims

1. Gram-negative mutant of a mucosal bacterium, comprising a mutation such that it is viable, is capable of OMP formation and lacks endotoxic lipopolysaccharide (LPS), the mutant being free of LPS.

2. Gram-negative mutant according to claim 1 comprising a mutation such that it is free of Lipid A.

3. Gram-negative mutant according to claim 1 or 2, said bacterium being selected from the group comprising diplococci.

4. Gram-negative mutant according to any one of claims 1-3, said bacterium being selected from the group comprising gonococci, e.g. *N. gonorrhoeae.*

5. Gram-negative mutant according to any one of claims 1-3, said bacterium being selected from the group comprising meningococci e.g. *N*. *meningitidis.*

6. Gram-negative mutant according to claim 1 or 2, said bacterium being selected from the group comprising *Rordetella,* e.g. *Bordetella pertussis.*

7. Gram-negative mutant according to any on of the preceding claims, said mutant comprising a mutation in at least one gene associated with Lipid A biosynthesis.

8. Gram-negative mutant according to any one of the preceding claims, said mutant comprising a mutation in at least one gene associated with the early stage of Lipid A biosynthesis, said early stage being prior to formation of the following structure

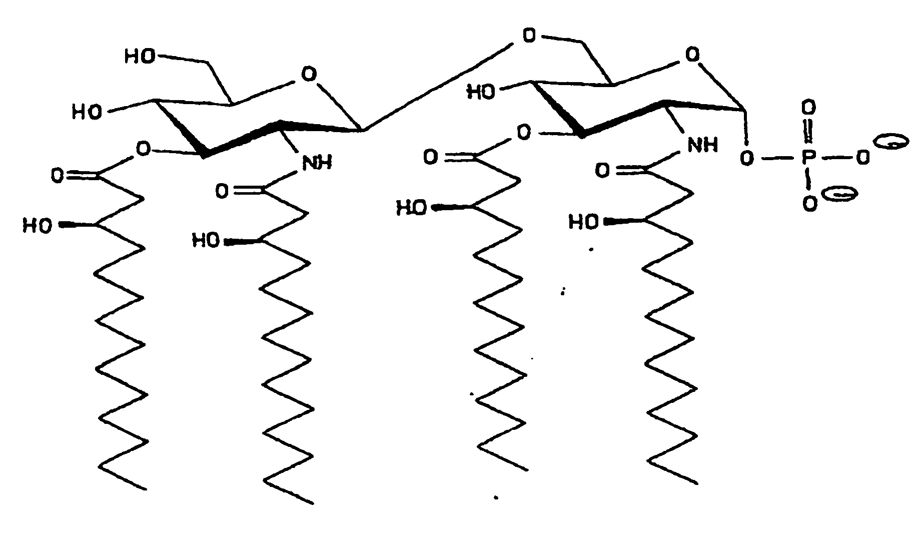

9. Gram-negative mutant according to any one of the preceding claims, said mutant comprising a mutation in at least one gene detected from the group comprising *lpxA. lpxD* and *lpxB.*

10. Gram-negative mutant according to any one of the preceding claims, said mutant comprising a mutation in at least the gene *lpxA.*

11. Attenuated live vaccine against a gram-negative mucosal bacterium, said vaccine comprising a mutant according to any one of claims 1-10 as an active component and a pharmaceutically acceptable carrier.

12. Whole cell vaccine against a gram-negative mucosal bacterium, said vaccine comprising a mutant according to any one of claims 1-10 as an active component and a pharmaceutically acceptable carrier.

13. Vaccine according to any one of claims 11-12, said vaccine being substantially free of endotoxic LPS, wherein substantially free is defined as LPS-free according to the Limulus assay.

14. Outer membrane vesicle vaccine against a Gram-negative mucosal bacterium, said vaccine comprising outer membrane vesicles derived from a mutant according to any one of claims 1-10, and a pharmaceutically acceptable carrier, said vaccine being substantially free of endotoxic LPS, wherein substantially free is defined as LPS-free according to the Limulus assay.

15. Outer membrane vesicle vaccine according to claim 14, said outer membrane vesicle comprising an OMP.

16. Vaccine according to any one of claims 11-15 further comprising an adjuvant.

17. A method of producing LPS-free vacine, the method comprising the steps of:
a) culturing a mutant according to any one of claims 1-10; and,
b) mixing the mutant or outer membrane vesicles derived from the mutant, with a pharmaceutically acceptable carrier.

18. A method according to claim 17, wherein the outer membrane vesicles comprise an OMP.

19. A method of producing LPS-free OMP, the method comprising the steps of:
a) culturing a mutant according to any one of claims 1-10; and,
b) isolating OMP from said culture.

## Patentansprüche

1. Gramnegative Mutante eines Mucosabakteriums, das eine Mutation aufweist, so daß sie lebensfähig ist, zur OMP-Bildung in der Lage ist und ihr ein endotoxisches Lipopolysaccharid (LPS) fehlt, wobei die Mutante frei von LPS ist.

2. Gramnegative Mutante nach Anspruch 1,
die eine Mutation aufweist, so daß sie frei von Lipid A ist.

3. Gramnegative Mutante nach Anspruch 1 oder 2,
wobei das Bakterium aus der Gruppe ausgewählt ist, die Diplokokken aufweist.

4. Gramnegative Mutante nach einem der Ansprüche 1 bis 3,
wobei das Bakterium aus der Gruppe ausgewählt ist, die Gonokokken, z.B. N. gonorrhoeae, aufweist.

5. Gramnegative Mutante nach einem der Ansprüche 1 bis 3,
wobei das Bakterium aus der Gruppe ausgewählt ist, die Meningokokken, z.B. N. meningitidis, aufweist.

6. Gramnegative Mutante nach Anspruch 1 oder 2,
wobei das Bakterium aus der Gruppe ausgewählt ist, die Bordetella, z.B. Bordetella pertussis, aufweist.

7. Gramnegative Mutante nach einem der vorstehenden Ansprüche,
wobei die Mutante eine Mutation in mindestens einem Gen aufweist, das mit der Biosynthese von Lipid A verbunden ist.

8. Gramnegative Mutante nach einem der vorstehenden Ansprüche,
wobei die Mutante eine Mutation in mindestens einem Gen aufweist, das mit der frühen Stufe der Biosynthese von Lipid A verbunden ist, wobei dieses frühe Stufe vor der Bildung der folgenden Struktur liegt:

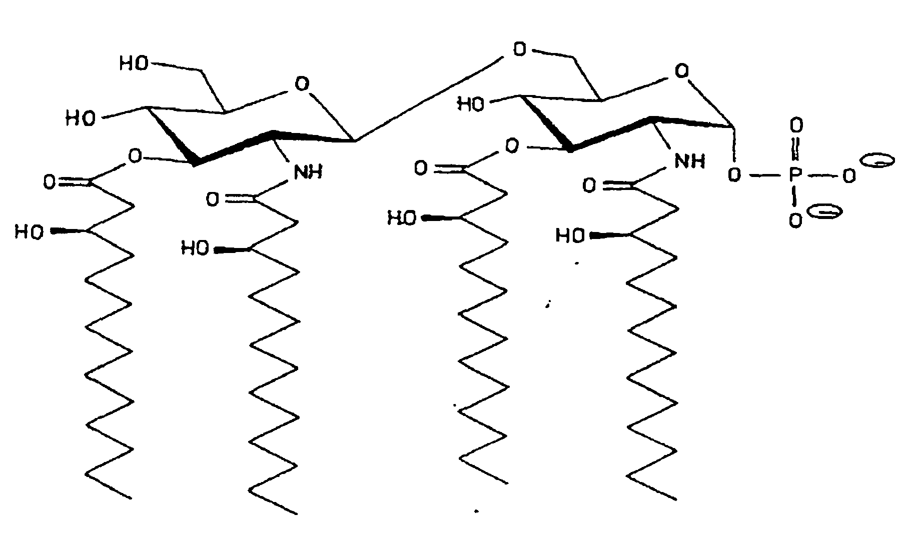

9. Gramnegative Mutante nach einem der vorstehenden Ansprüche,
wobei die Mutante eine Mutation in mindestens einem Gen aufweist, das aus der Gruppe ausgewählt ist, die lpxA, lpxD und lpxB aufweist.

10. Gramnegative Mutante nach einem der vorstehenden Ansprüche,
wobei die Mutante eine Mutation zumindest in dem Gen IpxA aufweist.

11. Attenuierter Lebendimpfstoff gegen ein gramnegatives Mucosabakterium,
wobei der Impfstoff eine Mutante nach einem der Ansprüche 1 bis 10 als einen Wirkstoff und einen pharmazeutisch akzeptablen Träger aufweist.

12. Vollzellimpfstoff gegen ein gramnegatives Mucosabakterium,
wobei der Impfstoff eine Mutante nach einem der Ansprüche 1 bis 10 als einen Wirkstoff und einen pharmazeutisch akzeptablen Träger aufweist.

13. Impfstoff nach einem der Ansprüche 11 und 12,
wobei der Impfstoff im wesentlichen frei von endotoxischem LPS ist, wobei im wesentlichen frei als LPS-frei gemäß dem Limulus-Assay definiert ist.

14. Impfstoff aus äußeren Membran-Vesikeln gegen ein gramnegatives Mucosabakterium,
wobei der Impfstoff äußere Membran-Vesikel, die von einer Mutante nach einem der Ansprüche 1 bis 10 stammen, und einen pharmazeutisch akzeptablen Träger aufweist,
wobei der Impfstoff im wesentlichen frei von endotoxischem LPS ist, wobei im wesentlichen frei als LPS-frei gemäß dem Limulus-Assay definiert ist.

15. Impfstoff aus äußeren Membran-Vesikeln nach Anspruch 14,
wobei das äußere Membran-Vesikel ein OMP aufweist.

16. Impfstoff nach einem der Ansprüche 11 bis 15, der ferner ein Adjuvans aufweist.

17. Verfahren zum Herstellen eines LPS-freien Impfstoffs,
wobei das Verfahren die folgenden Schritte aufweist:
a) Züchten einer Mutante nach einem der Ansprüche 1 bis 10; und
b) Mischen der Mutante oder von äußeren Membran-Vesikeln, die von dieser Mutante stammen, mit einem pharmazeutisch akzeptablen Träger.

18. Verfahren nach Anspruch 17,
wobei die äußeren Membran-Vesikel ein OMP aufweisen.

19. Verfahren zum Herstellen von LPS-freiem OMP,
wobei das Verfahren die folgenden Schritte aufweist:
a) Züchten einer Mutante nach einem der Ansprüche 1 bis 10; und
b) Abtrennen des OMP aus der Kultur.

## Revendications

1. Mutant gram négatif d'une bactérie des muqueuses, comprenant une mutation telle qu'il est viable, capable de formation de protéines de la membrane externe (OMP) et ne possède pas le lipopolysaccharide (LPS) endotoxique, le mutant étant dépourvu de LPS.

2. Mutant gram négatif selon la revendication 1, comprenant une mutation telle qu'il est dépourvu de lipide A.

3. Mutant gram négatif selon la revendication 1 ou 2, ladite bactérie étant choisie dans le groupe comprenant les diplocoques.

4. Mutant gram négatif selon l'une quelconque des revendications 1 à 3, ladite bactérie étant choisie dans le groupe comprenant les gonocoques, par exemple *N*. *gonorrhoeae.*

5. Mutant gram négatif selon l'une quelconque des revendications 1 à 3, ladite bactérie étant choisie dans le groupe comprenant les méningocoques, par exemple *N*. *meningitidis.*

6. Mutant gram négatif selon la revendication 1 ou 2, ladite bactérie étant choisie dans le groupe comprenant *Bordetella,* par exemple *Bordetella* pertussis.

7. Mutant gram négatif selon l'une quelconque des revendications précédentes, ledit mutant comprenant une mutation dans au moins un gène associé à la biosynthèse du lipide A.

8. Mutant gram négatif selon l'une quelconque des revendications précédentes, ledit mutant comprenant une mutation dans au moins un gène associé au stade précoce de la biosynthèse du lipide A, ledit stade précoce précédant la formation de la structure suivante

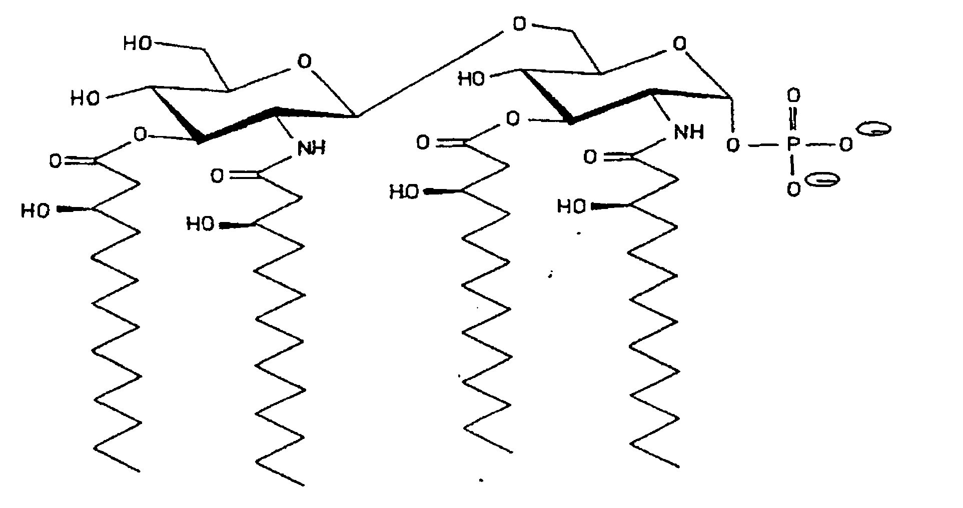

9. Mutant gram négatif selon l'une quelconque des revendications précédentes, ledit mutant comprenant une mutation dans un moins un gène choisi dans le groupe constitué par *lpxA, lpxD* et *lpxB.*

10. Mutant gram négatif selon l'une quelconque des revendications précédentes, ledit mutant comprenant une mutation au moins dans le gène *lpxA.*

11. Vaccin vivant atténué contre une bactérie des muqueuses Gram négatif, ledit vaccin comprenant un mutant selon l'une quelconque des revendications 1 à 10 en tant qu'un composant actif et un vecteur pharmaceutiquement acceptable.

12. Vaccin à cellules entières contre une bactérie des muqueuses Gram négatif, ledit vaccin comprenant un mutant selon l'une quelconque des revendications 1 à 10 en tant qu'un composant actif et un vecteur pharmaceutiquement acceptable.

13. Vaccin selon l'une quelconque des revendications 11 et 12, ledit vaccin étant substantiellement dépourvu de LPS endotoxique, dans lequel substantiellement dépourvu est défini comme dépourvu de LPS selon le test de Limulus.

14. Vaccin à base de vésicules de la membrane externe contre une bactérie des muqueuses Gram négatif, ledit vaccin comprenant des vésicules de la membrane externe dérivées d'un mutant selon l'une quelconque des revendications 1 à 10, et un vecteur pharmaceutiquement acceptable, ledit vaccin étant substantiellement dépourvu de LPS endotoxique, dans lequel substantiellement dépourvu est défini comme dépourvu de LPS selon le test de Limulus.

15. Vaccin à base de vésicules de la membrane externe selon la revendication 14, ladite vésicule de la membrane externe comprenant une OMP.

16. Vaccin selon l'une quelconque des revendications 11 à 15, comprenant en outre un adjuvant.

17. Procédé de production d'un vaccin dépourvu de LPS, le procédé comprenant les étapes consistant à:
a) cultiver un mutant selon l'une quelconque des revendications 1 à 10 ; et
b) mélanger le mutant ou les vésicules de la membrane externe dérivées du mutant, avec un vecteur pharmaceutiquement acceptable.

18. Procédé selon la revendication 17, dans lequel les vésicules de la membrane externe comprennent une OMP.

19. Procédé de production d'une OMP dépourvue de LPS, le procédé comprenant les étapes consistant à:
a) cultiver un mutant selon l'une quelconque des revendications 1 à 10 ; et
b) isoler l'OMP de ladite culture.


### SEQUENCE LISTING

(1) GENERAL INFORMATION:
   (i) APPLICANT:
      (A) NAME: De Staat der Nederlanden
      (B) STREET: P.O. box 1
      (C) CITY: Bilthoven
      (D) STATE: Utrecht
      (E) COUNTRY: The Netherlands
      (F) POSTAL CODE (ZIP): 3720 BA
   (ii) TITLE OF INVENTION: Novel mutants of gramnegative mucosal bacteria and application thereof in vaccines.
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0. Version #1.25 (EPO)
(2) INFORMATION FOR SEQ ID NO: 1:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: E. coli
   (xi) SEQUENCE DESCRIPTION: SEQ ID NO: 1:
      ACTGACGCGT GTGATTGATA AATCCGC 27
(3) INFORMATION FOR SEQ ID NO: 2:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: E. coli
   (xi) SEQUENCE DESCRIPTION: SEQ ID NO: 2:
      GTAGGGCGGC ACGTCCTGCG CCACACCGGA 30
(4) INFORMATION FOR SEQ ID NO: 3:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: N. meningitidis
   (xi) SEQUENCE DESCRIPTION: SEQ ID NO: 3:
      TCCGGTGTGG CGCAGGACGT GCCGCCCTAC 30
(5) INFORMATION FOR SEQ ID NO: 4:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: N. meningitidis
   (xi) SEQUENCE DESCRIPTION: SEQ ID NO: 4:
      CGGCCGCTCT AGAACTAGTG GATCA 25